(19) Europäisches Patentamt
European Patent Office
Office européen des brevets

(11) EP 2 724 713 A1

(12) **EUROPÄISCHE PATENTANMELDUNG**

(43) Veröffentlichungstag:
**30.04.2014 Patentblatt 2014/18**

(51) Int Cl.:
***A61K 8/73*** *(2006.01)* ***A61K 8/81*** *(2006.01)*
***A61Q 5/06*** *(2006.01)*

(21) Anmeldenummer: **12190209.2**

(22) Anmeldetag: **26.10.2012**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**

(71) Anmelder: **BASF SE**
**67056 Ludwigshafen (DE)**

(72) Erfinder:
- **Patwardhan-Huber, Darshan**
  **67117 Limburgerhof (DE)**
- **Gemba, Bruno**
  **67678 Mehlingen (DE)**

(74) Vertreter: **Reinhardt, Jürgen**
**BASF**
**Personal Care and Nutrition GmbH**
**Henkelstraße 67**
**40589 Düsseldorf (DE)**

(54) **Klare, transparente Haargele**

(57) Vorgeschlagen wird eine Zusammensetzung, enthaltend
a) Chitosan und/oder Chitosanderivate und/oder Chitosansalze und
b) ein Copolymer bestehend aus quervernetztem N-Vinylpyrrolidon, N-Vinyl-imidazol, 3-Methyl-1-Vinylimidazoliumchlorid und Methacrylsäure ,
die Verwendung dieser Zusammensetzung zur Herstellung klarer, transparenter Haargele, sowie das daraus resultierende Haargel.

EP 2 724 713 A1

## Beschreibung

### Gebiet der Erfindung

Die Erfindung befindet sich auf dem Gebiet der Haarkosmetik und betrifft eine Kombination von kationischen Verdickern und Chitosansalzen sowie deren Verwendung zur Herstellung klarer, transparenter Haargele und die haarkosmetischen Zubereitungen an sich.

### Stand der Technik

Haarstylingmittel sind ein wesentlicher Bestandteil der heute im kosmetischen Markt nachgefragten Produkte. Haarkosmetische Zusammensetzungen mit frisurfestigenden Eigenschaften werden in Form von Aerosolschäumen, Haarsprays oder Haargelen angeboten.

Die Anforderungen an diese Produkte sind umfangreich. Mit dem Ziel, den ästhetischen Anforderungen gerecht zu werden, sollen Haarstylingmittel dem Haar einerseits eine langanhaltende stabile Form geben, dennoch aber ausreichende Elastizität und Flexibilität des Haares gewährleisten, um das natürliche Aussehen und den angenehmen Griff des Haares zu erhalten. Die behandelten Haare sollen sich nicht klebrig anfühlen oder sogar brüchig sein. Außerdem sollte die Haarkosmetik möglichst leicht auskämmbar sein ohne plaqueartige Polymerreste zu hinterlassen, die den Eindruck von Schuppen erwecken könnten. Vorteilhafterweise haben diese Formulierungen neben den ästhetischen Aspekten auch konditionierende pflegende Eigenschaften.

Derzeit beobachtet man einen Trend zu stärker festigenden Stylingmitteln. Unter den Haarstylingmitteln erfreuen sich Haargele zunehmender Beliebtheit auch beim männlichen Kundenkreis, häufig begründet in dem geringen Zeitaufwand bei der Anwendung.

Das Auftragen der Stylingmittel in Form von Haargelen setzt jedoch eine ausreichende Viskosität der Formulierung voraus. Erhöht man allerdings den Anteil an viskositätssteigernden Polymeren erhöht sich das Risiko der Plaque- und Flockenbildung (Flaking-Effekt) beim Brechen des Filmes oder beim Auskämmen, was einerseits einen negativen ästhetischen Eindruck hinterlässt und andererseits auch zur Austrocknung und Brüchigkeit der Haare beiträgt. Zusätzlich zieht ein höherer Anteil dieser wasserlöslichen Polymere aufgrund ihrer Hydrophilität auch eine erhöhte Anfälligkeit der Frisur gegen Luftfeuchte nach sich mit der Folge einer verringerten Wellstabilität. Besonders bei hoher Luftfeuchtigkeit lässt die festigende Wirkung sehr schnell nach und das Haar kehrt in seine ursprüngliche Form zurück. Diese Eigenschaft wird mit der sogenannten Wellstabilität (curl-retention) gemessen, bei der behandelte vorher in Locken geformte Haare über einen definierten Zeitraum einer bestimmten Luftfeuchte ausgesetzt werden und die Länge der Locken im Verhältnis zum ursprünglichen Längengrad bestimmt wird.

Ein weiteres Problem bei der Formulierung von Haargelen besteht in der Herstellung klarer transparenter Gele, die üblicherweise in Haarstylingmitteln eingesetzten Polymere ergeben häufig milchige Gele. So wird gerade beim Einsatz des häufig in Stylingmitteln verwendeten kationischen Polymers Chitosan, das konditionierende Eigenschaften hat, die Formulierung insbesondere bei pH-Werten größer 6 trüb. Das Auftragen eines Haargels mit den Händen auf die Haare erfordert es jedoch, dass auch die Formulierung ein ästhetisches Aussehen hat, da ein milchiges Erscheinungsbild beim Anwender den Eindruck hinterlässt, dass die Formulierung im Haar deutlich sichtbar sein könnte.

Übliche wesentliche Inhaltsstoffe von Haargelen sind Verdickerpolymere, filmbildende konditionierende Polymere und kationische Polymere.

Polymere kationische Verdicker mit Monomereinheiten aus N-Vinylimidazol, Methacrylaten, N-vinylpyrrolidon oder N-Vinylcaprolactam sind als Rheologiemodifizierungsmittel für haarkosmetische Zubereitungen insbesondere Haargele aus der Internationalen Anmeldung WO 200710035 A1 bereits bekannt.

Die Europäische Anmeldung EP 1 075 832 A1 beschreibt die Kombination von kationischen Polymeren mit einem Terpolymer aus Vinylpyrrolidon, Vinylcaprolactam und einem basischen Acrylamidmonomer zur Anwendung in Haarbehandlungsmitteln als Stylingmittel. Jedoch werden mit der vorgeschlagenen Kombination nur Aerosol- und Pumpschaumfestiger, sowie Pump-Sprühlotionen hergestellt. Auch die deutsche Patentschrift DE 4343378 C1 hat konditionierende Haarpflegemittel in Form von Aerosolschaum-Formulierungen zum Inhalt, die eine Kombination von kationischen Polymeren, quaternären Ammoniumverbindungen mit Chitosanderivaten und -salzen darstellen. Der Zusatz von Chitosanderivaten oder -salzen zu PVP/VA Copolymeren vermindert die Spannungsrissbildung der auf das Haar gebrachten Filme **(**WO 97/02007**).**

Die Internationale Anmeldung WO 2012/055665 A2 offenbart eine Kombination eines Copolymers aus N-Vinylpyrrolidon, N-Vinylimidazol, N-Methyl-N'-vinylimidazoliumchlorid und Methacrylsäure mit einem Polyol mit mindestens drei Hydroxygruppen zur Verformung von Haaren und Haarpflege bevorzugt in Form eines Haargels.

Aufgabe der vorliegenden Erfindung war es, klare transparente Haargele zur Verfügung zu stellen, die sich durch verbesserte rheologische Eigenschaften mit Auswirkungen auf die sensorischen Eigenschaften der damit behandelten Haare (gutes Anfaßgefühl, Griff, Volumen, Handhabbarkeit) auszeichnen. Die mit den Gelen behandelten Haare

sollen eine gute Steifheit und auch bei erhöhter Luftfeuchtigkeit eine gute Wellstabilität (curl-retention) aufweisen. Die Formulierung soll sich dennoch sauber auskämmen und einfach auswaschen lassen.

## Beschreibung der Erfindung

Gegenstand der Erfindung, mit der diese Aufgaben gelöst wurden, ist eine Zusammensetzung, enthaltend

a) Chitosan und/oder Chitosanderivate und/oder Chitosansalze und
b) ein Copolymer bestehend aus quervernetztem N-Vinylpyrrolidon, N-Vinylimidazol, 3-Methyl-1-Vinylimidazoliumchlorid und Methacrylsäure

Die erfindungsgemäße Zusammensetzung enthält die Komponenten a) und b) im Gewichtsverhältnis 1 : 1 bis 1:10, vorzugsweise im Gewichtverhältnis 1:1 bis 1:3.
Mit einer Kombination der Komponenten a) und b) ist es möglich transparente klare Haargele mit ausreichend hoher Viskosität herzustellen, die sich durch eine gute Wellstabilität (Curl-Retention) auch bei hoher Luftfeuchte auszeichnet und keine Plaquebildung beim Auskämmen zeigt.
Ein weiterer Gegenstand der Erfindung ist ein klares transparentes Haargel, enthaltend

a) Chitosan und/oder Chitosanderivate und/oder Chitosansalze und
b) ein Copolymer bestehend aus quervernetztem N-Vinylpyrrolidon, N-Vinyl-imidazol, 3-Methyl-1-Vinylimidazoliumchlorid und Methacrylsäure

Die erfindungsgemäßen Haargele enthalten

a) 0,3 bis 3 Gew. %, vorzugsweise 0,5 bis 2,0 Gew., besonders bevorzugt 0,5 bis 1,0 Gew. % Chitosan und/oder Chitosanderivate und/oder Chitosansalze und

b) 0,5 bis 3 Gew. %, vorzugsweise 1,0 bis 2,5 Gew. % eines Copolymers bestehend aus quervernetztem N-Vinylpyrrolidon, N-Vinylimidazol, 3-Methyl-1-Vinylimidazoliumchlorid und Methacrylsäure bezogen auf das Gewicht des Haargels.

### Klare transparente Haargele

Das erfindungsgemäße Mittel liegt in Form eines Gels, welches vorzugsweise direkt mit der Hand aufgetragen wird, in Form einer viskosen Lotion oder in Form eines Sprühgels vor, das mit einer mechanischen Vorrichtung versprüht wird.
Unter klaren transparenten Haargelen werden im Rahmen der vorliegenden Erfindung Gele verstanden, die auf der Handfläche mit bloßem Auge klar erscheinen (handklar).
Diese Gele haben bei einer Transmissionsmessung bei 600nm und Raumtemperatur einen Transmissionswert von > 93%.
Als Träger für die klaren, transparenten Haargele, sowie Lösungs-und Quellmittel für die festen Komponenten wird Wasser oder Alkohol/Wasser-Mischungen eingesetzt. Geeignete Alkohole sind Ethanol, Methanol, Isopropanol und/oder n-Propanol, wobei Ethanol und isoPropanol zu den bevorzugten Alkoholen zählen. Die Träger enthalten mindestens 10 Gew. % Wasser, vorzugsweise mindestens 60 Gew.%, besonders bevorzugt mindestens 80 Gew. % Wasser bezogen auf den Träger.
Die Viskosität des Gels beträgt 5.000 bis 200.000 mPas, vorzugsweise 10.000 bis 70.000 mPas und besonders bevorzugt von 20.000 bis 60.000 mPas und insbesondere 40.000 bis 55.000 mPas (Viskosität Brookfield DV-II +, bei 25°C).
Der pH-Wert des Gels liegt im leicht sauren, gut hautverträglichen Bereich von pH 3 bis 7,4, bevorzugt im Bereich von pH 4,0 bis 6,0.
Ein weiterer Vorteil der Kombination von Chitosan, Chitosanderivaten und/oder -salzen (Komponte a)) als guten Filmbildner mit konditionierenden Eigenschaften und dem Copolymer bestehend aus quervernetztem N-Vinylpyrrolidon, N-Vinylimidazol, 3-Methyl-1-Vinylimidazoliumchlorid (Komponente b)) besteht darin dass Chitosan in der Kombination mit diesem kationischen Verdicker nicht zur Ausfällung kommt und es somit möglich ist klare, transparente Gele auch mit der Komponente a) herzustellen.
Bei der Herstellung von Gelen auf Basis der erfindungsgemäßen Polymerkombination a) und b) können als weitere Gelbildner übliche Komponenten eingesetzt werden, beispielsweise, um spezielle rheologische oder andere anwendungstechnische Eigenschaften der Gele einzustellen. Weitere Verdickungsmittel werden gegebenenfalls in einer

Menge von 0,05 bis 10, bevorzugt von 0,1 bis 2 Gew.% eingesetzt, so dass die Gele eine Viskosität von mindestens 5000 mPas aufweisen.

Als Gelbildner eignen sich leicht vernetzte Polyacrylsäure, beispielsweise Carbomer (INCI), Cellulosederivate, z. B. Hydroxypropylcellulose, Hydroxyethylcellulose, kationisch modifizierte Cellulosen, Polysaccharide, z. B. Xanthangummi, Capryl/Caprin-Triglycerid, Natriumacrylat-Copolymere, Polyquatemium-32 (und) Paraffinum Liquidum (INCI), Natriumacrylat-Copolymere (und) Paraffinum Liquidum (und) PPG-1 Trideceth-6, Acrylamidopropyltrimoniumchlorid/Acrylamid-Copolymere, Steareth-10 Allylether Acrylat-Copolymere, Polyquatemium-37 (und) Paraffinum Liquidum (und) PPG-1 Trideceth-6, Polyquatemium 37 (und) Propylenglycoldicapratdicaprylat (und) PPG-1 Trideceth-6, Polyquatemium-7, Polyquatemium-44. Geeignete vernetzte Homopolymere der Acrylsäure sind beispielsweise kommerziell unter dem Namen Carbopol® (Noveon) erhältlich. Bevorzugt sind auch hydrophob modifizierte vernetzte Polyacrylat Polymere, wie Carbopol®Ultrez 21 (Noveon). Besonders geeignete Polymere sind Copolymere aus (Meth)acrylsäure und Polyetheracrylaten, wobei die Polyetherkette mit einem C8-C30-Alkylrest terminiert ist. Dazu zählen z. B. Acrylat/Beheneth-25-methacrylat-Copolymere, die als Aculyn® (Rohm und Haas) kommerziell erhältlich sind.

Chitosan und/oder Chitosanderivate und/oder Chitosansalze - Komponente a)

Chitosane werden zur Gruppe der Hydrokolloide gezählt. Im Gegensatz zu den meisten Hydrokolloiden, die im Bereich biologischer pH-Werte negativ geladen sind, stellen Chitosane unter diesen Bedingungen kationische Biopolymere dar.

Die positiv geladenen Chitosane aus N-Acetyl-D-Glucosamin und D-Glucosamin - Einheiten mit basischer Aminogruppe können mit entgegengesetzt geladenen Oberflächen in Wechselwirkung treten und werden daher in kosmetischen Haar- und Körperpflegemitteln z.B. auch als Filmbildner eingesetzt.

Chemisch betrachtet handelt es sich um partiell deacetylierte Chitine unterschiedlichen Molekulargewichtes, die den folgenden - idealisierten - Monomerbaustein enthalten:

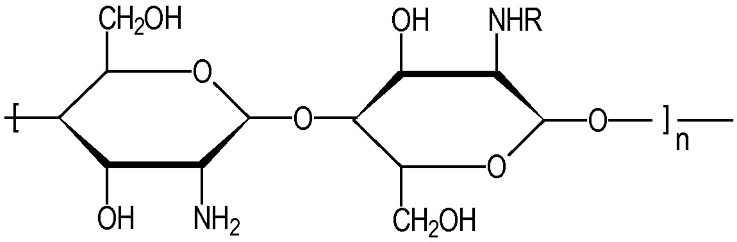

Der Deacetylierungsgrad beträgt zwischen 10 und 99 %, vorzugsweise 65 bis 99 %, besonders bevorzugt wird für die erfindungsgemäße Zubereitung ein Deacetylierungsgrad von über 80 %.

Zur Herstellung der Chitosane geht man von Chitin, vorzugsweise den Schalenresten von Krustentieren aus, die als billige Rohstoffe in großen Mengen zur Verfügung stehen. Das Chitin wird dabei in einem Verfahren das erstmals von Hackmann et al. beschrieben worden ist, üblicherweise zunächst durch Zusatz von Basen deproteiniert, durch Zugabe von Mineralsäuren demineralisiert und schließlich durch Zugabe von starken Basen deacetyliert, wobei die Molekulargewichte über ein breites Spektrum verteilt sein können.

In einer bevorzugten Ausführungsform der Erfindung werden besonders aschearme kationische Biopolymere eingesetzt, die man erhält, indem man

(a) frische Krustentierschalen mit verdünnter wässriger Mineralsäure behandelt,

(b) das resultierende demineralisierte erste Zwischenprodukt mit wässriger Alkalihydroxidlösung behandelt,

(c) das resultierende geringfügig deproteinierte zweite Zwischenprodukt erneut mit verdünnter wäßriger Mineralsäure behandelt,

(d) das resultierende decalcifizierte dritte Zwischenprodukt schließlich mit konzentrierter wäßriger Alkalilauge behandelt und dabei bis zu einem Gehalt von 0,05 bis 0,5 und insbesondere 0, 15 bis 0.25 Mol Acetamid pro Mol Monomereinheit deacetyliert, und

(e) gegebenenfalls einer Druck/Temperaturnachbehandlung zur Einstellung der Viskosität unterwirft.

Üblicherweise werden Chitosane mit einem mittleren Molekulargewicht von 10.000 bis 5.000.000 Dalton eingesetzt. Im kosmetischen Bereich kommen Chitosane mit unterschiedlichen durchschnittlichen Molekulargewichten zum Einsatz beispielsweise im niedermolekularen Bereich von 30.000 bis 100.000 Dalton, oder mit einem Molekulargewicht von 100.000 bis 1.000.000 Dalton sowie im höhermolekularen Bereich von 800.000 bis 1.200.000 Dalton. In der vorliegenden Erfindung werden insbesondere Chitosane mit einem Molekulargewicht von 50.000 bis 1000.000 Dalton bevorzugt. Diese weisen eine Viskosität nach Brookfield (1 Gew.-%ig in Glycolsäure) unterhalb von 5000 mPas, einen Deacetylierungsgrad im Bereich von 80 bis 88 % und einem Aschegehalt von weniger als 0,3 Gew.-% auf.

Werden die freien Aminogruppen des Chitosans mit Säuren neutralisiert, erhält man die entsprechenden Salze, die erfindungsgemäß besonders geeignet sind, da sie eine bessere Löslichkeit in Wasser oder wässrig-alkoholischen Lösungen aufweisen.

Für die Neutralisation geeignete Säuren sind organische Säuren ausgewählt aus der Gruppe, die gebildet wird von Milchsäure, Glykolsäure, Glutaminsäure, Essigsäure, Weinsäure, Ameisensäure, Malonsäure, Benzoesäure, Adipinsäure, Nitrilotriessigsäure, Zitronensäure, p-Hydroxybenzoesäure, Ethylendiaminteraessigsäure, Salicylsäure, Chlorsulfonsäure, Pyrrolidoncarbonsäure, Nicotinsäure, Hydroxyisobuttersäure und Hydroxyisovaleriansäure, sowie anorganische Säuren wie beispielsweise Salzsäure oder Salpetersäure.

Vorzugsweise werden Salze der Milchsäure, Essigsäure, Ameisensäure oder Glykolsäure eingesetzt, besonders bevorzugt sind Salze der Milchsäure.

Die einzusetzende Säuremenge sollte zur vollständigen Neutralisation der freien Aminogruppen ausreichend sein, ein geringfügiger Säureüberschuss ist möglich.

Zu den Chitosanderivaten gehören Chitosane, die an den Glucanketten Seitengruppen tragen. Im Sinne der Erfindung zählen dazu anionisch, nichtionisch oder kationisch derivatisierte Chitosane, wie Carboxylierungs-, Succinylierungs-, Alkoxylierungs- oder Quatemierungsprodukte, die beispielsweise in der deutschen Patentschrift DE 3713099 C2 sowie der deutschen Patentanmeldung DE 19604180 A1 beschrieben werden.

<u>Copolymers bestehend aus quervernetztem N-Vinylpyrrolidon, N-Vinylimidazol, 3-Methyl-1 - Vinylimidazoliumchlorid und Methacrylsäure</u>

Das in der erfindungsgemäßen Zusammensetzung eingesetzte Copolymer bestehend aus quervernetztem N-Vinylpyrrolidon, N-Vinylimidazol, 3-Methyl-1-Vinylimidazoliumchlorid und Methacrylsäure (Komponente b)) befindet sich unter dem Handelsnamen Luvigel® Advanced vertrieben durch die BASF SE im Handel, die INCI-Bezeichnung ist Polyquatemium-86

<u>PVP und/oder PVA-Copolymere</u>

Als weitere Komponente in Haargelen eignen sich nichtionische filmbildende und haarfestigende Polymere, die Homopolymere des Vinylpyrrolidons darstellen und unter dem Handelsnamen Luviskol® K von der BASF SE vertrieben werden. Auch Polyvinylpyrrolidon/VinylacetatCopolymere (PVP/VA), die bekannte Schutzkolloide für kosmetische Anwendungen darstellen haben sich als zusätzliche Komponente c) in den erfindungsgemäßen Gelen sehr bewährt, sie sind beispielsweise unter der Marke Luviskol® VA von der BASF SE Ludwigshafen/FRG erhältlich sind. Üblicherweise werden die Copolymere als wäßrige Lösungen mit einem Feststoffgehalt von 1,5 bis 30 Gew.% eingesetzt. Die klaren, transparenten Gele enthalten 1 bis 20 Gew. %, bevorzugt 5 bis 15 Gew. % und besonders bevorzugt 12 bis 13 Gew. % der PVP-und/oder PVA-Copolymere bezogen auf das Gewicht der klaren transparenten Gele.

<u>Methoden</u>

Bestimmung der Biegesteifigkeit (Stiffness):

Die Festigung von polymeren Filmbildnern wurde außer durch subjektive Beurteilung (Handtest) auch physikalisch durch Messung der Biegesteifigkeit von dünnen Haarsträhnen (jeweils ca. 3 g und 24 cm Länge) gemessen. Dazu wurden die gewogenen, trockenen Haarsträhnen in die 3,0 Gew.-%ige Polymerlösung (Lösungsmittel: Ethanol/Wasser 55:45 w/w) getaucht, wobei durch dreimaliges Eintauchen und Herausnehmen und anschließendes Ausdrücken zwischen Filterpapier eine gleichmäßige Benetzung der Haarsträhne und Verteilung der Polymerlösung sichergestellt wurde. Die überschüssige Filmbildnerlösung wurde dann zwischen Daumen und Zeigefinger abgestreift und die Haarsträhnen wurden die Strähnen von Hand so geformt, dass sie einen etwa runden Querschnitt erhielten. Bei 21°C und 65 % relativer Feuchte wurde über Nacht im Klimaraum getrocknet. Die Prüfungen wurden im Klimaraum bei ca. 21°C und ca. 65 % relativer Feuchte mittels eines Zug/Druck-Prüfgerätes durchgeführt. Die Haarsträhne wurde symmetrisch an den Enden auf zwei zylindrische Rollen der Probenaufnahme gelegt. Genau in der Mitte wurde die Strähne dann von oben mit einem abgerundetem Stempel ca.40 mm durchgebogen (Brechen des Polymerfilms). Die dafür erforderliche Kraft (Fmax) wurde mit einer Wägezelle (50 N) bestimmt. Dabei stellt ein Messwert das arithmetische Mittel aus den Einzelmessungen an 5 bis 10 gleich behandelten Haarsträhnen dar. Die so ermittelten Werte wurden zu denen eines handelsüblichen Vergleichspolymers (Amphomer®LV-71) ins Verhältnis gesetzt und in % angegeben.

Bestimmung der Wellstabilität (Curl-Retention):

Für die Bestimmung der Wellstabilität wurden Haarsträhnen von ca. 2 g Gewicht und 15,5 cm Länge und aus mittelbraunem, kaukasischem Menschenhaar verwendet.

Behandlung der Haarsträhnen:

Die Haarsträhnen wurden zweimal mit einer wässrigen Texapon®NSO-Lösung gewaschen.
Anschließend wurden die Haarsträhnen mit warmem Wasser ausgespült, bis keine Schaumbildung mehr erkennbar war und mit VE-Wasser nachgespült.

Die feuchte Haarsträhne wurde dann zwischen Filterpapier ausgedrückt, in die Polymerlösung (siehe oben) eingetaucht, mit den Fingern abgestreift und wieder zwischen Filterpapier ausgedrückt. Dieser Prozess wurde insgesamt dreimal durchgeführt.
Danach wurde das Haar um einen Teflonstab von 12 mm Durchmesser gewickelt und mit Filterpapier und Gummiring befestigt. Anschließend wurden die Haarsträhnen ca. 90 min bei 70 - 80°C im Wärmeschrank getrocknet. Nach Abkühlen auf Raumtemperatur wurden die Locken unter Erhalt der Form abgestreift und an einem Gestell aufgehängt und die Lockenlänge (L0) bestimmt.
Für die Bestimmung eines Curl-Retention-Wertes wurden 5 Haarlocken verwendet. Die Locken wurden hängend in eine Klimakammer mit 25°C und 90 % rel. Luftfeuchte gestellt. Nach 5 Stunden wurde die Länge (Lt) abgelesen.
Die Curl Retention errechnet sich wie folgt:

$$\text{Curl Retention in \%} = \frac{L - Lt}{L - Lo} * 100$$

L = Länge der Haare (15,5 cm)
L0 = Länge der Haarlocke nach dem Trocknen
Lt = Länge der Haarlocke nach Klimabehandlung

Als Curl Retention wird der Mittelwert aus den 5 Einzelmessungen nach 5 h bei 20°C und 90% rel. Feuchte angegeben.

<u>Weitere Hilfsstoffe</u>

Haarkonditioniermittel

Trotz der konditionierenden Wirkung des Chitosan(derivates) und/oder -salzes können die erfindungsgemäßen Zubereitungen 0,01 bis 20, bevorzugt von 0,05 bis 10, besonders bevorzugt von 0,1 bis 5 Gew.% eines weiteren Konditioniermittels enthalten.
Erfindungsgemäß bevorzugte Konditionierungsmittel sind beispielsweise alle Verbindungen, welche in der EP-A 934 956 (S.11-13) unter "water soluble conditioning agent" und "oil soluble conditioning agent" aufgeführten Verbindungen. Weitere vorteilhafte Konditionierungsmittel stellen beispielsweise die nach INCI als Polyquatemium bezeichneten Verbindungen dar (insbesondere Polyquaternium-1 bis Polyquaternium-56).
Zu den geeigneten Konditionierungsmitteln zählen beispielsweise auch polymere quaternäre Ammoniumverbindungen, kationische Cellulosederivate, Chitosanderivate und Polysaccharide. Das Konditioniermittel ist bevorzugt ausgewählt aus Betain, Panthenol, Panthenylethylether, Sorbitol, Proteinhydrolysaten, Pflanzenextrakten; A-B-Block-Copolymeren aus Alkylacrylaten und Alkylmethacryaten; A-B-Block-Copolymeren aus Alkylmethacrylaten und Acrylnitril; AB-A-Block-Copolymeren aus Lactid und Ethylenoxid; A-B-A-Block-Copolymeren aus Caprolacton und Ethylenoxid; A-B-C-Block-Copolymeren aus Alkylen- oder Alkadienverbindungen, Styrol und Alkylmethacrylaten; A-B-C-Block-Copolymeren aus Acrylsäure, Styrol und Alkylmethacrylaten, sternförmigen Block-Copolymeren, hyperverzweigten Polymeren, Dendrimeren, intrinsisch elektrisch leitfähigen 3,4-Polyethylendioxythiophenen und intrinsisch elektrisch leitfähigen Polyanilinen.
Weitere erfindungsgemäß vorteilhafte Konditioniermittel stellen Cellulosederivate und quaternisierte Guargum

Derivate, insbesondere Guar Hydroxypropylammoniumchlorid (z.B. Jaguar Excel®, Jaguar C 162® (Rhodia), CAS 65497-29-2, CAS 39421-75-5) dar.

Auch nichtionische Poly-N-vinylpyrrolidon/Polyvinylacetat-Copolymere (z.B. Luviskol®VA 64 (BASF)), anionische Acrylat-Copolymere (z.B. Luviflex®Soft (BASF)), und/oder amphotere Amid/Acrylat/Methacrylat Copolymere (z.B. Amphomer® (National Starch)) können erfindungsgemäß vorteilhaft als Konditioniermittel eingesetzt werden.

Hydrotrope

Zur Verbesserung des Fließverhaltens können ferner Hydrotrope, wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Typische Beispiele sind

- Glycerin;
- Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methylolverbindungen, wie insbesondere Trimethylolethan, Trimetylolpropan, Trimetylolbutan, Pentaerythrit und Dipentaerythrit;
- Niedrigalkylglucoside, insbesondere solche mit 1 bis 8 Kohlenstoffen im Alkylrest, wie beispielsweise Methyl- und Butylglucosid;
- Zuckeralkohole mit 5 bis 12 Kohlenstoffatomen, wie beispielsweise Sorbit oder Mannit;
- Zucker mit 5 bis 12 Kohlenstoffatomen, wie beispielsweise Glucose oder Saccharose;
- Aminozucker, wie beispielsweise Glucamin.

Öle, Fette und Wachse

Die erfindungsgemäßen kosmetischen, bevorzugt haarkosmetischen Zubereitungen können auch Öle, Fette oder Wachse enthalten, soweit die Transparenz des Haargels dadurch nicht beeinträchtigt wird. Dieses ist unter anderem von dem Verhältnis Alkohol/Wasser des Trägers beeinflusst. Öle, Fette oder Wachse werden vorteilhaft gewählt aus der Gruppe der Lecithine und der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, wie z.B. Olivenöl, Sonnenblumenöl, Sojaöl, Erdnußöl, Rapsöl, Mandelöl, Palmöl, Kokosöl, Rizinusöl, Weizenkeimöl, Traubenkemöl, Distelöl, Nachtkerzenöl, Macadamianußöl und dergleichen mehr. Weitere polare Ölkomponenten können gewählt werden aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat Dicaprylyl Carbonat (Cetiol CC) und Cocoglyceride (Myritol 331), Butylen Glycol Dicaprylat/Dicaprat und Dibutyl Adipat sowie synthetische, halbsynthetische und natürliche Gemische solcher Ester, wie z.B. Jojobaöl.

Ferner können eine oder mehrere Ölkomponenten vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen. Erfindungsgemäß vorteilhaft sind Mischungen aus C12-15-Alkylbenzoat

und 2-Ethylhexylisostearat, Mischungen aus C12-15-Alkylbenzoat und Isotridecylisononanoat sowie Mischungen aus C12-15-Alkylbenzoat, 2-Ethylhexylisostearat und Isotridecylisononanoat. Vorteilhaft wird die Ölphase gewählt aus der Gruppe 2-Ethylhexylisostearat, Octyldodecanol, Isotridecylisononanoat, Butylen Glycol Dicaprylat/Dicaprat, 2-Ethylhexylcocoat, $C_{12-15}$-Alkylbenzoat, Capryl-Caprin-säure-triglycerid, Dicaprylylether.

Besonders vorteilhaft sind Mischungen aus Octyldodecanol, Capryl-Caprinsäure-triglycerid, Dicaprylylether, Dicaprylyl Carbonat, Cocoglyceriden oder Mischungen aus $C_{12-15}$-Alkylbenzoat und 2-Ethylhexylisostearat, Mischungen aus $C_{12-15}$-Alkylbenzoat und Butylen Glycol Dicaprylat/Dicaprat sowie Mischungen aus $C_{12-15}$-Alkylbenzoat, 2-Ethylhexylisostearat und Isotridecylisononanoat.

Von den Kohlenwasserstoffen sind Paraffinöl, Cycloparaffin, Squalan, Squalen, hydriertes Polyisobuten bzw. Polydecen vorteilhaft im Sinne der vorliegenden Erfindung zu verwenden.

Die Ölkomponente wird ferner vorteilhaft aus der Gruppe der Phospholipide gewählt. Als erfindungsgemäß vorteilhaftes Paraffinöl kann erfindungsgemäß Merkur®Weissoel Pharma 40 von Merkur Vaseline, Shell Ondina® 917, Shell Ondina®927, Shell Oil 4222, Shell Ondina®933 von Shell & DEA Oil, Pionier® 6301 S, Pionier® 2071 (Hansen & Rosenthal) eingesetzt werden.

Der Gehalt an Ölen, Fetten und Wachsen beträgt höchstens 20, bevorzugt höchstens 10, weiter bevorzugt höchstens 5 Gewichts-%, bezogen auf das Gesamtgewicht der Zusammensetzung.

Erfindungsgemäße Zubereitungen

Zu den erfindungsgemäßen Zubereitungen zählen klare, transparente Haargele enthaltend:

a) 0,3 bis 3 Gew. % Chitosan und/oder Chitosanderivate und/oder Chitosansalze und
b) 0,5 bis 3 Gew. % eines Copolymers bestehend aus quervernetztem N-Vinyl-pyrrolidon, N-Vinylimidazol, 3-Methyl-1-Vinylimidazoliumchlorid und Methacrylsäure bezogen auf das Gewicht des Haargels,

bevorzugt

a) 0,5 bis 2 Gew. % Chitosan und/oder Chitosanderivate und/oder Chitosansalze und
b) 0,5 bis 3 Gew. % eines Copolymers bestehend aus quervernetztem N-Vinyl-pyrrolidon, N-Vinylimidazol, 3-Methyl-1-Vinylimidazoliumchlorid und Methacrylsäure bezogen auf das Gewicht des Haargels, oder

a) 0,5 bis 2 Gew. % Chitosan und/oder Chitosanderivate und/oder Chitosansalze und
b) 0,5 bis 3 Gew. % eines Copolymers bestehend aus quervernetztem N-Vinyl-pyrrolidon, N-Vinylimidazol, 3-Methyl-1-Vinylimidazoliumchlorid und Methacrylsäure und
c) 1 bis 20 Gew. % eines nichtionischen Polymers aus der Gruppe der PVP- und/oder PVA-Copolymere bezogen auf das Gewicht des Haargels,

besonders bevorzugt

a) 0,5 bis 1 Gew. % Chitosan und/oder Chitosanderivate und/oder Chitosansalze und
b) 1 bis 2,5 Gew. % eines Copolymers bestehend aus quervernetztem N-Vinyl-pyrrolidon, N-Vinylimidazol, 3-Methyl-1-Vinylimidazoliumchlorid und Methacrylsäure bezogen auf das Gewicht des Haargels, oder

a) 0,5 bis 1 Gew. % Chitosan und/oder Chitosanderivate und/oder Chitosansalze und
b) 1 bis 2,5 Gew. % eines Copolymers bestehend aus quervernetztem N-Vinyl-pyrrolidon, N-Vinylimidazol, 3-Methyl-1-Vinylimidazoliumchlorid und Methacrylsäure
c) 5 bis 15 Gew. % eines nichtionischen Polymers aus der Gruppe der PVP- und/oder PVA-Copolymere

bezogen auf das Gewicht des Haargels und
speziell bevorzugt

a) 0,5 bis 1 Gew. % Chitosan und/oder Chitosanderivate und/oder Chitosansalze und
b) 1 bis 2,5 Gew. % eines Copolymers bestehend aus quervernetztem N-Vinyl-pyrrolidon, N-Vinylimidazol, 3-Methyl-1-Vinylimidazoliumchlorid und Methacrylsäure und
c) 12 bis 13 Gew. % eines nichtionischen Polymers aus der Gruppe der PVP- und/oder PVA-Copolymere bezogen auf das Gewicht des Haargels.

**Beispiele**

Beispiel 1

Herstellung der Haargele:

Phase A wurde durch Verrühren der Komponenten (solubilisieren) hergestellt. Danach wurden die Komponenten der Phase B in Phase A eingewogen und bis zur Homogenität gerührt.
Schließlich wurde Phase C in die Lösung aus den Phasen A und B eingerührt.

| | | V1 | | V2 | | Vergleich | |
|---|---|---|---|---|---|---|---|
| **500 g** | | **Ansatz** | % | **Ansatz** | % | **Ansatz** | % |
| **Phase A** | | | | | | | |
| Perfum | | 0,2 | 0,10 | 0,2 | 0,10 | 0,2 | 0,10 |
| Cremophor CO 40 | PEG-40 Hydrogenated Castor Oil | 0,6 | 0,30 | 0,6 | 0,30 | 0,6 | 0,30 |
| Wasser dem. | Aqua dem. | 146 | 73,00 | 144,2 | 72,10 | 146,2 | 73,10 |
| **Phase B** | | | | | | | |
| Hydagen HCMS-LS | Chitosanlactat | 0,2 | 0,10 | 2 | 1,00 | - | - |
| Luviskol K 85 CQ-Lösung | PVP | 24 | 12,00 | 24 | 12,00 | 24 | 12,00 |
| Luviquat Supreme | Polyquaternium-68 | 10 | 5,00 | 10 | 5,00 | 10 | 5,00 |
| Panthenol 50P | | 3 | 1,50 | 3 | 1,50 | 3 | 1,50 |
| 1,2 Propylenglykol Care | Propylen Glycol | 10 | 5,00 | 10 | 5,00 | 10 | 5,00 |
| Euxyl PE 9010 | Phenoxyethanol, Ethylhexylglycerin | 1 | 0,50 | 1 | 0,50 | 1 | 0,50 |
| **Phase C** | | | | | | | |
| Luvigel Advanced | Polyquaternium-86 | 5 | 2,50 | 5 | 2,50 | 5 | 2,50 |
| | | 200,00 | 100,00 | 200,00 | 100,00 | 200,00 | 100,00 |
| | | | | | | | |
| Aussehen [visuell] | | fast klar leicht gelblich | | fast klar leicht gelblich | | fast klar leicht gelblich | |
| Struktur | | 2 - 3 | | 2 | | 2 | |
| pH | | 5,7 | | 5,7 | | 5,6 | |
| Viskosität Brookfield DV-II +, [mPas] | | 30200 [Sp.6/20 upm] | | 52600 [Sp.7/20 upm] | | 31200 [Sp.6/20 upm] | |
| Stiffness* cN (20°C / 65% rel. Humity) | | 250 ± 24 | | 419 ± 40 | | 225 ± 36 | |
| curl-retention nach 5 h; (25°C / 90% rel. Feuchte) [%] | | 83 | | 91 | | 85 | |
| curl-retention nach 24 h; (25°C / 90% rel. Feuchte) [%] | | 74 | | 88 | | 72 | |

* getestete Gele, (50:170 verdünnt mit Wasser)

| | | Vergleich | | V3 | | V4 | |
|---|---|---|---|---|---|---|---|
| | | **Ansatz** | % | **Ansatz** | % | **Ansatz** | % |
| **Phase A** | | | | | | | |
| Perfum | | 0,2 | 0,10 | 0,2 | 0,10 | 0,2 | 0,10 |
| Cremophor CO 40 | PEG-40 Hydrogenated Castor Oil | 0,6 | 0,30 | 0,6 | 0,30 | 0,6 | 0,30 |
| Wasser dem. | Aqua dem. | 171,2 | 85,60 | 170,2 | 85,10 | 169,2 | 84,60 |
| **Phase B** | | | | | | | |
| Hydagen HCMS-LS | | - | - | 1 | 0,50 | 2 | 1,00 |
| Luviskol K 90 Lösung ca. 20% | PVP | 25 | 12,50 | 25 | 12,50 | 25 | 12,50 |
| Euxyl PE 9010 | Phenoxyethanol, Ethylhexylglycerin | 1 | 0,50 | 1 | 0,50 | 1 | 0,50 |
| **Phase C** | | | | | | | |
| Luvigel Advanced | Polyquaternium-86 | 2 | 1,00 | 2 | 1,00 | 2 | 1,00 |
| | | 200,00 | 100,00 | 200,00 | 100,00 | 200,00 | 100,00 |
| | | | | | | | |
| Aussehen [visuell] | | fast klar leicht gelblich | | fast klar leicht gelblich | | fast klar leicht gelblich | |
| Struktur | | 1-2 | | 1-2 | | 1-2 | |
| pH | | 5,2 | | 5,2 | | 5,2 | |
| Viskosität Brookfield DV-II +, [mPas] | | 27600 [Sp.6/20upm] | | 31400 [Sp.6/20upm] | | 39600 [Sp.6/20upm] | |
| Stiffness* cN (20°C / 65% rel. Humity) | | 185 ± 24 | | | | 295 ± 28 | |
| curl-retention nach 5 h; (25°C / 90% rel. Feuchte) [%] | | 80 | | | | 88 | |

* getestete Gele, (50:170 verdünnt mit Wasser)

Bei Zugabe von 0,5 oder 1,0 Gew. % Chitosan zum Copolymer Luvigel Advanced erfolgt eine deutliche synergistische Zunahme der Viskosität und insbesondere eine Verbesserung der Curl-Retention bei hoher Luftfeuchte.

Aus den vorgelegten Versuchen ist ersichtlich, dass Versuch V 2 die Kombination aus

a) 1 Gew. % Chitosan und/oder Chitosanderivaten und/oder Chitosansalzen und

b) 2,5 Gew. % eines Copolymers bestehend aus quervernetztem N-Vinyl-pyrrolidon, N-Vinylimidazol, 3-Methyl-1-Vinylimidazoliumchlorid und Methacrylsäure besonders gute Werte hinsichtlich der Wellstabilität aufweist.

## Patentansprüche

**1.** Zusammensetzung, enthaltend

a) Chitosan und/oder Chitosanderivate und/oder Chitosansalze und

b) ein Copolymer bestehend aus quervernetztem N-Vinylpyrrolidon, N-Vinyl-imidazol, 3-Methyl-1-Vinylimidazoliumchlorid und Methacrylsäure

**2.** Zusammensetzung nach Anspruch 1, enthaltend die Komponenten a) und b) im Gewichtsverhältnis 1 : 1 bis 1:10

**3.** Verwendung einer Zusammensetzung gemäß den Ansprüchen 1 und/oder 2 zur Herstellung transparenter klarer Haargele.

4. Klare transparente Haargele, enthaltend

a) Chitosan und/oder Chitosanderivate und/oder Chitosansalze und
b) ein Copolymer bestehend aus quervernetztem N-Vinylpyrrolidon, N-Vinyl-imidazol, 3-Methyl-1-Vinylimidazoliumchlorid und Methacrylsäure

5. Klare transparente Haargele, nach Anspruch 4, **dadurch gekennzeichnet, dass** sie

a) 0,3 bis 3 Gew. % Chitosan und/oder Chitosanderivate und/oder Chitosansalze
und
b) 0,5 bis 3 Gew. % eines Copolymers bestehend aus quervernetztem N-Vinyl-pyrrolidon, N-Vinylimidazol, 3-Methyl-1-Vinylimidazoliumchlorid und Methacrylsäure
bezogen auf das Gewicht des Haargels enthalten.

6. Klare transparente Haargele, nach den Ansprüchen 4 und/oder 5, **dadurch gekennzeichnet, dass** sie als weitere Komponente c) PVP und/oder PVA-Copolymere enthalten.

7. Klare transparente Haargele, nach den Ansprüchen 4 bis 6, **dadurch gekennzeichnet, dass** sie die Komponente c) in einer Menge von 1 bis 20 Gew. % bezogen auf die Menge des Haargels enthalten.

8. Klare transparente Haargele, nach mindestens einem der Ansprüche 1 bis 3, **dadurch gekennzeichnet, dass** als Komponente a) Chitosansalze eingesetzt werden.

9. Klare transparente Haargele, nach mindestens einem der Ansprüche 1 bis 3, **dadurch gekennzeichnet, dass** als Komponente a) Chitosanlactat eingesetzt wird.

Europäisches Patentamt
European Patent Office
Office européen des brevets

# EUROPÄISCHER RECHERCHENBERICHT

Nummer der Anmeldung
EP 12 19 0209

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | WO 2012/084400 A2 (HENKEL AG & CO KGAA [DE]; MUELLER BURKHARD [DE]; KAFTAN PAMELA [DE]) 28. Juni 2012 (2012-06-28)<br>* Seite 1, Absätze 1, 4 *<br>* Seite 2, Absätze 2, 3 *<br>* Seite 12, Absatz 6 *<br>* Seite 16, Absatz 6 *<br>* Ansprüche *<br>----- | 1-9 | INV.<br>A61K8/73<br>A61K8/81<br>A61Q5/06 |
| Y | DE 10 2008 030660 A1 (HENKEL AG & CO KGAA [DE]) 7. Januar 2010 (2010-01-07)<br>* Seite 2, Absatz 0001 *<br>* Seite 4, Absatz 0023 *<br>* Ansprüche *<br>----- | 1-9 | |
| Y | DE 10 2008 030661 A1 (HENKEL AG & CO KGAA [DE]) 7. Januar 2010 (2010-01-07)<br>* Seite 2, Absatz 0001 *<br>* Seite 4, Absatz 0026 *<br>* Ansprüche *<br>----- | 1-9 | RECHERCHIERTE SACHGEBIETE (IPC)<br>A61K<br>A61Q |
| Y | DE 198 05 434 A1 (WELLA AG [DE]) 12. August 1999 (1999-08-12)<br>* Seite 2, Zeile 24ff *<br>* Beispiele *<br>* Ansprüche *<br>----- | 1-9 | |
| Y | DE 10 2010 043016 A1 (HENKEL AG & CO KGAA [DE]) 3. Mai 2012 (2012-05-03)<br>* Seite 1, Absatz 0001 *<br>* Seite 2, Absätze 0005, 0006 *<br>* Seite 16, Absatz 0084 *<br>* Beispiele *<br>----- | 1-9 | |

Der vorliegende Recherchenbericht wurde für alle Patentansprüche erstellt

1

| Recherchenort | Abschlußdatum der Recherche | Prüfer |
|---|---|---|
| München | 13. März 2013 | Heller, Dorothée |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
..........................................
& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR. EP 12 19 0209

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

13-03-2013

| Im Recherchenbericht angeführtes Patentdokument | Datum der Veröffentlichung | Mitglied(er) der Patentfamilie | Datum der Veröffentlichung |
|---|---|---|---|
| WO 2012084400 A2 | 28-06-2012 | DE 102010055814 A1 | 28-06-2012 |
| | | WO 2012084400 A2 | 28-06-2012 |
| DE 102008030660 A1 | 07-01-2010 | DE 102008030660 A1 | 07-01-2010 |
| | | EP 2451530 A2 | 16-05-2012 |
| | | WO 2010000607 A2 | 07-01-2010 |
| DE 102008030661 A1 | 07-01-2010 | DE 102008030661 A1 | 07-01-2010 |
| | | EP 2291169 A1 | 09-03-2011 |
| | | WO 2010000608 A1 | 07-01-2010 |
| DE 19805434 A1 | 12-08-1999 | DE 19805434 A1 | 12-08-1999 |
| | | EP 0943312 A2 | 22-09-1999 |
| | | JP 11255628 A | 21-09-1999 |
| | | US 6264929 B1 | 24-07-2001 |
| DE 102010043016 A1 | 03-05-2012 | DE 102010043016 A1 | 03-05-2012 |
| | | WO 2012055665 A2 | 03-05-2012 |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- WO 200710035 A1
- EP 1075832 A1
- DE 4343378 C1
- WO 9702007 A
- WO 2012055665 A2
- DE 3713099 C2
- DE 19604180 A1
- EP 934956 A